# EUROPEAN PATENT APPLICATION

(11) **EP 3 444 592 A1**
(43) Date of publication of application: **20.02.2019**
(21) Application number: 18189071.6
(22) Date of filing: 15.08.2018
(51) Int. Cl.: G01N 21/77, G01N 35/00

(54) **METHOD FOR SAMPLE ANALYSIS IN AN AUTOMATIC ANALYSER**

(30) Priority: 16.08.2017 LU 100369
(71) Applicant: Stratec Biomedical AG, 75217 Birkenfeld-Graefenhausen (DE)
(72) Inventor: BIRKNER, Nico, 75217 Birkenfeld (DE)
(74) Representative: Tegethoff, Sebastian

(57) **Abstract**

The invention relates to a foil and its use in a method for analyzing a sample in an automatic analyzer, comprising the steps of providing foil comprising at least one immobilized reagent; forming the foil to a receptacle; adding the sample to the receptacle; and analyzing a reaction between sample and immobilized reagent.

## Description

### Field of the invention

The field of the invention relates to a method for sample analysis in an automatic analyser.

### Background of the invention

A variety of assays are routinely used in clinical diagnostics and life sciences. The assay components comprise a sample and at least one reagent and are usually stored in separate receptacles apart from each other. To carry out an assay, the assay components are brought together in a reaction vessel. In general, the transfer of assay components into a reaction vessel is carried out by a pipettor in an automatic analyser.

In standard liquid clinical diagnostic assays such as immuno-assays or molecular testing, the reagents and the sample are loaded on the automatic analyser in separate transport and/ or storing receptacles. The reagents and the sample are then pipetted from their transport or storing receptacle into a reaction vessel. Single cuvettes or manifold receptacles such as micro titer and multi well plates are often used as reaction vessels. For a specific assay, manufacturers prefabricate specific kits comprising various reagents. To work through an assay, the automatic analyser needs to be loaded with the sample, the reagents and the consumables such as pipette tips and reaction vessels.

Paper based lateral flow tests are also very common and include pregnancy and drogue stripe tests. In paper based lateral flow tests, a sample is applied onto a paper stripe comprising immobilized indicator reagents. The sample is applied with or without a fluxing agent and dried immuno-conjugates are often used as indicator reagents. The immuno-conjugates are dissolved by an added liquid, which can be the sample, and react with the analytes to be detected in the sample. The liquid travels across the test stripe and analytes concentrate at the respective immuno-conjugate indicator spot. Depending on the approach, a color change, fluorescence, luminescence, staining or similar effect manifests itself at the enriching spot.

Published US-patent application No. US 2017/067881 discloses such paper microfluidic devices for the detection of bodily fluids. The devices can be used, for example, for detection of bodily fluids from or at crime scenes, including blood, saliva, semen, urine, feces, vaginal fluids, and perspiration. Detection can be performed using colorimetric reagents that react when placed in contact with the fluid of interest. A single device can be used to test for multiple bodily fluids at the same time. The devices of US 2017/067881 A1 are not suitable for use in automated analyzer systems, in particular in the context of complex immunoassays.

Different microfluidic approaches for miniaturized detection reactions are available on the market. Therein, microfluidic chambers are generated with screen or ink printing technology, wherein liquid carrying ducts are generated on paper by printing specific areas with hydrophobic or hydrophilic material. At the same time reagents can be printed and later be brought together with the sample in a different way than pipetting them into the same reaction vessel. The microfluidic reaction vessel can further be modified for example with microfluidic chambers and printed sensors.

The publication of Focke and colleagues (Lab on a chip, Vol 10, No. 11, 2010) discloses functionalized microfluidic devices made of thin flexible films. The publication does not refer to the immobilization of reagents, but to their storage in such flexible microfluidic devices. The devices disclosed by Focke and colleagues have complex functionalities which make them not suitable for their use in an analyzer system due to their complexity.

Reis et a (Lab on a chip, Vol 16 No. 15, 2016) teach capillaries that are coated with a reagent. A liquid will enter such a capillary due to capillary forces. This document does not provide a receptacle into which liquids can be added, for example by pipetting.

It is a disadvantage of the prior art, that at least one uptake and one release step is necessary to bring the sample and/or the reagents to the reaction vessel, which often is a separate reaction vessel. In most of the cases the sample and the reagents need to be loaded on the analyser as liquids stored and/or transported in a receptacle. The sample and reagents are then brought together in a separate reaction vessel by a pipettor. Alternatively, the reagents come in a receptacle and the sample is added to that receptacle as a liquid.

### Object of the invention

It is an object of the invention to provide a method for sample analysis in an automatic analyser for simplification of the sample analysis process in an automatic analyzer.

### Summary of the invention

The instant invention provides a method for analyzing a sample in an automatic analyzer, comprising the steps of:
- providing foil comprising at least one immobilized reagent
- forming the foil to a receptacle;
- adding the sample to the receptacle; and
- analyzing a reaction between sample and immobilized reagent.

It is intended that the foil can be formed to a pipette tip, tube, cavity, bottle, well or multi-well.

In a further aspect of the invention, forming the foil may comprise a thermal deformation or a winding process.

It is further envisaged that at least one reagent can be immobilized onto the foil prior to providing the foil by drying a liquid , wherein immobilization of the reagent may be performed by printing the reagent onto the foil.

Analyzing a reaction within the meaning of the present disclosure may encompass detecting an immune, chemical or enzymatic reaction, wherein detecting a chemical or enzymatic reaction may comprise initiating an immune, fluorescence or a luminescence reaction.

In another aspect of the invention, adding a sample may be a result of using a foil formed to a pipette tip for transfer of the sample.

The sample can be added to a foil formed to a receptacle for carrying out the analysing step.

It is further intended that the method comprises the step of printing at least one hydrophilic and at least one hydrophobic area onto the foil, before forming the foil, wherein the hydrophobic area may be printed around the hydrophilic area.

The method may further comprise the step of providing a foil the sample area that is located at the tip of the receptacle and a reaction area comprising the reagent, wherein the sample area and the reaction area are connected by a channel and the reaction area is connected by a further channel to a waste area so that the sample can flow from the sample area to the reaction area further to the waste area.

Another object of the present disclosure is a foil comprising at least one immobilized reagent, wherein the foil can be made of a material allowing to form it into a receptacle.

It is intended that the foil may comprise at least one hydrophilic area and at least one hydrophobic areas, wherein the at least one hydrophobic area surrounds the at least one hydrophilic area.

A foil of the present disclosure may further comprise multiple hydrophilic areas that are connected by a channel.

The foil may further have a first hydrophilic area for taking up a sample and a second hydrophilic area which comprises the at least one immobilized reagent.

A third hydrophilic area may be used as a waste area for taking up residues from a chemical or enzymatic reaction within the second hydrophilic area.

The foil of the present disclosure may be formed to a pipette tip, tube, cavity, bottle, well or multi-well.

The use of a as described above in an automated analyzer system for analyzing samples is another object of the present invention.

### Summary of the figures

The invention will now be described on the basis of figures. It will be understood that the embodiments and aspects of the invention described in the figures are only examples and do not limit the protective scope of the claims in any way. The invention is defined by the claims and their equivalents. It will be understood that features of one aspect or embodiment of the invention can be combined with a feature of a different aspect or aspects of other embodiments of the invention. It shows:
- Figure 1: Top view schematic of a foil used in the method for sample analysis according to the first embodiment of the invention.
- Figure 2: Perspective view schematic of the foil of figure 1 formed to a tube.

### Detailed description of the invention

The invention provides a method for sample analysis in an automatic analyser enabling a simplified and customized sample analysis. The present invention does neither refer to a complex microfluidic device nor to a simple approach that is comparable to paper chromatography. The disclosed method can be implemented into automated analyzer systems for simplifying assays. The reagent to be immobilized can be chosen depending on the assay to be performed. Such assay can be immunoassays where it is necessary to use the specific reagents depending on the antigen to be detected or verified.

The term foil shall be understood within the meaning of the present disclosure as relating to a thin sheet or thin layer of a material that may be selected from the group of paper, metal, plastic, synthetic or biological prepared material, glass and aluminum. A foil represents a thin matrix of such materials that may be soaked or comprise a liquid.

An immobilized reagent within the meaning of the present invention comprises an immobilized solid, powder or liquid, also a dried liquid, comprising an analyte, buffer, reagent, solution, beads in solution and a mixture of liquid and solids. The immobilized reagent may also encompass a coating or a partial coating of the foil.

The words channel and drain are used synonymously for a liquid connection between two hydrophilic areas for instance.

A receptacle within the meaning of the present disclosure refers to a pipette tip, tube, cavity, bottle, well or multi-well which does not allow capillary forces to take effect.

A sample analysis process comprises the steps of providing consumables and at least one sample in a liquid state to the automatic analyser, bringing the sample together with at least one reagent, and analysing if at least one analyte in the sample reacts with the reagent.

The step of providing can be a loading process. The sample may comprise at least one analyte, wherein the analyte can react with at least one reagent in an enzymatic or chemical color change reaction or in a light emitting chemical or enzymatic reaction such as a luminescence or a fluorescence reaction, wherein a fluorescence reaction is a type of luminescence reaction characterized by emitting fluorescent light. The sample may further comprise a solvent, a fluxing agent, anticoagulant, preservative and/or a buffer. The reagents may be selected from the group comprising but not limited to: dried immuno-conjugates, enzymes, enzyme substrates, primers, nucleotides, dyes, DNA and/or RNA molecules comprising a quencher and/or a reporter and antibodies possibly linked to a reporter molecule or another reagent. Consumables may be selected from the group comprising but not limited to: solvents, glue, hydrophobic material, hydrophilic material, matrices, reagents, pipette tips, receptacles and reaction vessels such as cuvettes, tubes, multi well plates, glue.

Standard components pre-printed on a foil according to the invention shall be selected from the group comprising but not limited to: hydrophilic material, hydrophobic material, glue, fixing agents, and enzyme substrates. In a dissolving process bonds between elements of a compound to be solved are replaced by bonds to a solvent molecule.

The invention describes a method for sample analysis in an automatic analyser comprising the steps of providing a liquid sample and a foil comprising at least one immobilized reagent to the automatic analyser, forming the foil to a receptacle, pipette tip or tube or forming at least one cavity in the foil; bringing the at least one reagent in contact with a liquid sample, wherein the reagent is dissolved by the sample; and analyzing if at least one analyte in the sample reacts with the reagent. The forming step of the foil may be carried out in the automatic analyser in a thermal deformation or winding process. The foil may consist of a foil material on reels or foil material stripes. Alternatively, the foil in the providing step does not comprise the immobilized reagent or is pre-printed with standard components and the method further comprises an immobilizing step before the forming step, wherein at least one reagent is immobilized on the foil. The immobilization step may be achieved by printing the reagents onto the foil. The printing step can be carried out by standard inject printer's technology. The foil can for example be supplied in a flat form on a roll or in a cartridge in the providing step. It is new and preferred that the immobilization process takes place on-the-fly in the automatic analyser, because it enables the production of a customized foil. The foil may be pre-printed with standard components, so that only the desired reagents are printed onto the foil inside the automatic analyser. Alternatively, the immobilization process can take place outside the automatic analyser in an upstream production step.

In a first embodiment, the foil is further printed with hydrophobic and hydrophilic material so that it comprises at least one hydrophobic area surrounding at least one hydrophilic area. The hydrophilic area comprises at least one sample area, one reaction area and one waste area, wherein the sample area is connected to the reaction area by a first channel and the reaction area is connected to the waste area by a second channel and wherein the reaction area comprises immobilized reagents. After the immobilization process, the foil is formed to a tube and sample is applied to the sample area by immersing the sample area in the sample by dipping the tip of the tube on the respective side into the sample. Due to the hydrophilic and hydrophobic surface properties, respectively, the sample follows the hydrophilic path and automatically flows from the sample area over the first channel to the reaction area and further over the second channel to the waste area. Once arrived in the reaction area, the sample reacts with the reagent in a light emitting chemical or enzymatic reaction. The analyzing step can directly be carried out on the tube without using further reaction vessels or any other receptacle. Moreover, no pipettor, pipetting pump or pipette tips are necessary, if a picker arm is used to dip the tip of the foil into the sample. The number of different reactions can be varied by varying the number of reaction areas. The tube can be formed in a way that the reagents and the hydrophobic and hydrophilic areas are on the outside of the tube or on the inside of the tube. If the reagents and the hydrophilic and hydrophobic areas are on the outside of the tube or as soon as the tube diameter is big enough, it is convenient to have multiple hydrophilic areas each comprising a reaction area. If the reagents and the hydrophilic and hydrophobic areas are on the inside of the tube, the diameter of the tube needs to be big enough to avoid capillary forces and to avoid that a sample drop touches and thereby connects multiple reaction areas.

In a second embodiment, the foil is formed to a pipette tip, wherein the pipette tip can directly be used to take up the sample from the sample storing or sample transporting receptacle by aspiration. The formed pipette tip therefore functions as reaction vessel at the same time, wherein the reagents react with analyte in the sample to be detected. The analysing step can therefore be carried out inside the pipette tip or in a reaction vessel such as a cuvette or tube. It is new that a pipette tip can be used as reaction vessel.

In a third embodiment, the foil is formed to a reaction vessel and the reagents may be re-suspended by adding a solvent or another liquid before adding the sample. The analyzing step is then carried out in the formed reaction vessel, wherein the reaction vessel may have the form of cuvette.

In a fourth embodiment at least one reagent comprising cavity is formed into the foil. The sample is applied to at least one cavity, for example with a pipettor, where the reagents can react with an analyte in the sample.

The advantages of the invention of the present disclosure can be summarized as follows:
a. The invention allows to reduce the costs, loading effort and space by using less pipette tips and receptacles.
b. The invention allows to use a pipette tip as a reaction vessel
c. The invention allows to avoid extra pipetting steps.
d. The invention does not necessarily need a pipettor.
e. The invention allows for customization of the reagents immobilized on the foil in the automatic analyser.

### Detailed description of the figures

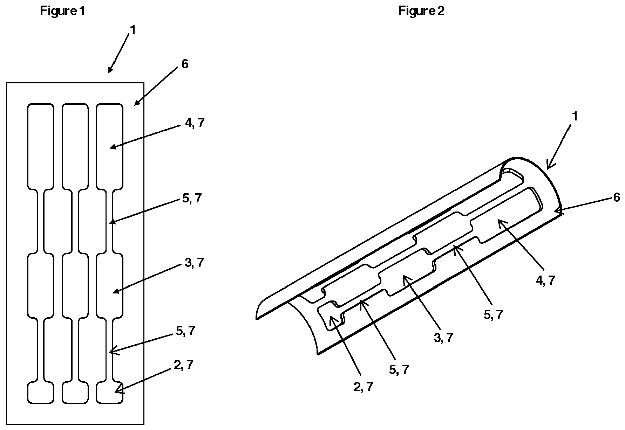

Figure 1 shows a top view of a foil 1 according to the first embodiment for carrying out three analyses, comprising three hydrophilic areas 7 surrounded by a hydrophobic area 6, wherein each hydrophilic area comprises a sample area 2 connected by a channel 5 to a reaction area 3 connected by a channel 5 to a waste area 4.
Figure 2 shows a perspective view of half of the assay foil of figure 1 formed to a tube.

### Reference Numerals

- 1: assay foil
- 2: sample area
- 3: reaction area
- 4: waste area
- 5: channel
- 6: hydrophobic area
- 7: hydrophilic area

## Claims

1. A method for analyzing a sample in an automatic analyzer, comprising the steps of:
a) providing foil comprising at least one immobilized reagent
b) forming the foil to a receptacle;
c) adding the sample to the receptacle; and
d) analyzing a reaction between sample and immobilized reagent.

2. The method according to claim 1, wherein a step of immobilizing the reagent onto the foil is performed into the same device that will subsequently perform steps a) to d) of claim 1.

3. The method according to claim 1 or 2, wherein the foil is formed to a pipette tip, tube, cavity, bottle, well or multi-well.

4. The method according to any one of claims 1 to 3, wherein forming the foil comprises a thermal deformation or a winding process.

5. The method according to any one of claims 1 to 4, wherein immobilization of the reagent is performed by printing the reagent onto the foil.

6. The method according to any one of claims 1 to 5, wherein analyzing a reaction comprises detecting an immune, chemical or enzymatic reaction.

7. The method according to claim 6, wherein detecting a chemical or enzymatic reaction comprises initiating an immune, fluorescence or a luminescence reaction.

8. The method according to any one of claims 1 to 7, wherein adding a sample is a result of using a foil formed to a pipette tip for transfer of the sample.

9. The method according to claim any one of claims 1 to 7, wherein the sample is added to a foil formed to a receptacle for carrying out the analyzing step.

10. The method according to any one of claims 1 to 9, further comprising the step of printing at least one hydrophilic and at least one hydrophobic area onto the foil, before forming the foil.

11. The method according to claim 10, comprising the step of printing the hydrophobic area around the hydrophilic

12. The method according to any one of claims 1 to 11, comprising the step of providing a foil at the sample area that is located at the tip of the receptacle and a reaction area comprising the reagent, wherein the sample area and the reaction area are connected by a channel and the reaction area is connected by a further channel to a waste area so that the sample can flow from the sample area to the reaction area further to the waste area.

13. A foil comprising at least one immobilized reagent, wherein the foil is made of a material allowing to form it into a receptacle.

14. The foil of claim 13, comprising at least one hydrophilic area and at least one hydrophobic areas, wherein the at least one hydrophobic area surrounds the at least one hydrophilic area.

15. The foil of any of claims 13 or 14, wherein multiple hydrophilic areas are connected by channel.

16. The foil of any one of claims 13 to 15, wherein a first hydrophilic area is intended for taking up a sample and a second hydrophilic area comprises the at least one immobilized reagent.

17. The foil of claim 16, wherein a third hydrophilic area is a waste area for taking up residues from a chemical or enzymatic reaction within the second hydrophilic area.

18. A foil of any one of claims 13 to 17, wherein the foil is formed to a pipette tip, tube, cavity, bottle, well or multi-well.

19. The use of a foil of any one of claims 13 to 18 in an automated analyzer system for analyzing samples.
